# EUROPÄISCHE PATENTSCHRIFT

(11) **EP 4 605 050 B1**
(45) Veröffentlichungstag und Bekanntmachung des Hinweises auf die Patenterteilung: **09.09.2026**
(21) Anmeldenummer: 23793779.2
(22) Anmeldetag: 19.10.2023
(51) Int. Cl.: A61M 16/08, A61M 16/22, A61M 16/20, A63B 23/18

(54) **VORRICHTUNG ZUR DURCHFÜHRUNG EINES HYPOXIE-TRAININGS**
DEVICE FOR PERFORMING HYPOXIA TRAINING
APPAREIL POUR EFFECTUER UN ENTRAÎNEMENT À L'HYPOXIE

(30) Priorität: 19.10.2022 DE 102022127597
(43) Veröffentlichungstag der Anmeldung: 27.08.2025
(73) Patentinhaber: Egorov, Egor, 10789 Berlin (DE)
(72) Erfinder: Egorov, Egor, 10789 Berlin (DE)
(74) Vertreter: Dantz, Dirk
(86) Internationale Anmeldenummer: PCT/EP2023/079152
(87) Internationale Veröffentlichungsnummer: WO 2024/083984

(56) Entgegenhaltungen:
- EP-A1- 3 912 665
- US-A1- 2016 095 994

## Beschreibung

Die Erfindung betrifft eine Vorrichtung zur Bereitstellung einer hypoxischen Gasmischung mit einem Gasreservoir, einer Gasabgabevorrichtung, einer Zuleitung, die dafür geeignet und dafür vorgesehen ist, eine Gasmischung vom Gasreservoir zur Gasabgabevorrichtung zu leiten, und/oder einer Ableitung, die dafür geeignet und dafür vorgesehen ist, eine Gasmischung von der Gasabgabevorrichtung zum Gasreservoir zu leiten und einem CO₂-Absorber, wobei der CO₂-Absorber in einem Trägerelement angeordnet ist. Die Offenbarung betrifft weiterhin ein Verfahren zur Durchführung eines Hypoxie-Trainings mit den Verfahrensschritten Einsetzen eines Behältnisses in ein Trägerelement einer Vorrichtung zur Bereitstellung einer hypoxischen Gasmischung, wobei das Behältnis einen CO₂-Absorber enthält, und Verbinden der Gasausgabevorrichtung mit Mund und/oder Nase des Nutzers. Die Offenbarung betrifft außerdem ein Behältnis mit einem CO₂-Absorber zur Durchführung eines Hypoxie-Trainings, wobei das Behältnis versiegelt ist.

### Stand der Technik

Die Hypoxie kann bei jeder Körperzelle Reaktionen hervorrufen und einen gesteigerten Energiestoffwechsel ermöglichen. Sie kann zur Aktivierung einer Vielzahl von Genen beitragen. Sportler, gesunde und kranke Menschen können von der Hypoxie profitieren.

Dass ein Höhentraining wirkt, ist schon lange Zeit bekannt. Aber wie der leichte Sauerstoffmangel zur Leistungsverbesserung im Körper führt, konnte man sich noch bis vor ein paar Jahren nicht ausreichend erklären. Die beobachtete Vermehrung der Erythrozyten reichte als Erklärung für die Veränderungen im Körper nicht aus. Den Durchbruch für das Verständnis brachte die Entdeckung des Hypoxie-induzierbaren Faktors HIF-1-alpha. Er lieferte die Erklärung für die umfassende Wirkung des Höhentrainings. Die Abkürzung HIF steht für Hypoxia-Inducible Factor. Hinter dem Fachbegriff verbirgt sich ein Sauerstoffsensor, der aktiv wird, wenn nicht genug Sauerstoff in den Körperzellen vorhanden ist. Er steuert einen der überlebenswichtigsten Prozesse im Körper: die Anpassung von Zellen, Geweben und Organen an einen Sauerstoffmangel. Gleichzeitig ist er ein Signal für die Selbstreparatur im Körper.

Die bekannteste positive Wirkung des HIF ist die Erythropoetinsynthese (EPO) in der Niere und Leber. Mit ihr erklärte man sich vor der Entdeckung von HIF die Veränderungen des Herz-Kreislauf-, Atmungs- und Blutsystems. Mittlerweile ist klar, dass die Leistungsverbesserung viel umfassender ist. Die Endothelzellen der Tunica intima reagieren auf den Einfluss der Hypoxie mit einer gesteigerten Stickstoffmonoxid-Synthese (NO). Das Gas beeinflusst entscheidend die Dilatation der Gefäße. Es verlässt das Endothel und bewirkt im umliegenden Gewebe eine Relaxation der glatten Muskelzellen. An der Tunica intima selbst verhindert NO die Adhäsion und Aggregation von Thrombozyten. Interessant ist in diesem Zusammenhang außerdem, dass die Endothelzellen unter dem Einfluss der Hypoxie den gefäßbildenden Faktor VEGF bilden. Durch seine Bildung kommt es im Rahmen der Hypoxie-Therapie zur einer Neoangiogenese der Kapillargefäße. Sehr häufig befinden sich diese zusätzlichen Gefäße in geschädigten oder minderdurchbluteten Gewebeabschnitten.

Alle bekannten Geräte zur Durchführung eines Hypoxie-Trainings verfügen über mindestens ein Atemreservoir, in das zu Beginn jeder diagnostischen und therapeutischen Sitzung eine bestimmte Menge atmosphärischer Luft zu Beginn jeder Diagnose-, Trainings- oder Therapiesitzung eingeleitet wird. Wenn der Benutzer, der an dieses Reservoir angeschlossen ist, aus diesem einatmet und ausatmet, wird der Sauerstoff in diesem Reservoir verbraucht, und es entsteht in diesem Reservoir ein Sauerstoff unterschüssiges sauerstoffhaltiges Gasgemisch. Dieses Gemisch wird von überschüssigem Kohlendioxid gereinigt, und und der Restsauerstoffgehalt wird darin zumindest periodisch während jeder Sitzung gemessen.

Die Schrift WO 2012/005712 stellt ein Gerät zum Atmen mit hypoxischen Gasgemischen vor bestehend aus: Tragrahmen; Atmungsbehälter mit Inspirationsventil, einem ersten Sensor für den Sauerstoffgehalt und einem Absorber für CO₂ und H₂O; einen steuerbaren Kompressor, der über ein Puffventil mit dem Atmungsbehälter verbunden ist; Kammer zur Entnahme und Akkumulation von aliquoten Gasgemische, ausgestattet mit einem zweiten Sensor für den Sauerstoffgehalt, steuerbarem Einstellventil und steuerbarem Ejektor; Benutzeraufsatz; eine Inspirationsleitung mit steuerbarem Zentralventil und erstem Durchflussmesser für die Inspirationsrate; ein T-Stück; eine Haupt-Exspirationsleitung, die mit einem mit einem ersten Luftgebläse ausgestattet ist und den Aufsatz mit dem Reservoir verbindet; zusätzlich Exspirationsleitung, die mit einem zweiten Durchflussmesser ausgestattet ist, der das Volumen der Durchflussmesser zur Messung des Volumens von Probenahmealiquoten und einem zweiten Luftgebläse ausgestattet ist und den Anschluss mit der Kammer verbindet; zusätzlicher Sensorkomplex und Steuereinheit.

Diese hier vorgestellte Vorrichtung ist sehr komplex aufgebaut, daher sehr teuer in der Anschaffung, die Anwendung ist insbesondere für einen privaten Nutzer sehr komplex und muss daher unter fachlicher Aufsicht stattfinden.

Es ist daher Aufgabe der vorliegenden Erfindung, eine Vorrichtung zur Bereitstellung einer hypoxischen Gasmischung bereitzustellen, die effizient, kostengünstig und sicher ein Hypoxie-Training für einen privaten Nutzer auch ohne fachliche Aufsicht ermöglicht.

Es wird weiterhin ein nicht beanspruchtes Verfahren zur Durchführung eines Hypoxie-Trainings offenbart, das eine sichere und gleichzeitig effiziente Durchführung eines Hypoxie-Trainings für einen privaten Nutzer auch ohne fachliche Aufsicht ermöglicht.

Es ist ebenfalls ein Behältnis mit einem CO2-Absorber zur Durchführung eines Hypoxie-Trainings offenbart, das eine sichere und gleichzeitig effiziente Durchführung eines Hypoxie-Trainings für einen privaten Nutzer auch nicht unter fachlicher Aufsicht ermöglicht.

Es ist daher Aufgabe der vorliegenden Erfindung, eine Vorrichtung zur Bereitstellung einer hypoxischen Gasmischung bereitzustellen, die effizient, kostengünstig und sicher ein Hypoxie-Training für einen privaten Nutzer auch nicht unter ärztlicher Aufsicht ermöglicht.

Weiterhin wird ein nicht beanspruchtes Verfahren zur Durchführung eines Hypoxie-Trainings offenbart, das eine sichere und gleichzeitig effiziente Durchführung eines Hypoxie-Trainings für einen privaten Nutzer auch ohne ärztliche Aufsicht ermöglicht.

Weiterhin wird ein Behältnis mit einem CO₂-Absorber zur Durchführung eines Hypoxie-Trainings offenbart, das eine sichere und gleichzeitig effiziente Durchführung eines Hypoxie-Trainings für einen privaten Nutzer auch nicht unter ärztlicher Aufsicht ermöglicht.

Die genannte Aufgabe wird mittels der Vorrichtung zur Bereitstellung einer hypoxischen Gasmischung gemäß Anspruch 1 gelöst. Weitere vorteilhafte Gestaltungen der Erfindung sind in den Unteransprüchen dargelegt.

Die erfindungsgemäße Vorrichtung zur Bereitstellung einer hypoxischen Gasmischung weist ein Gasreservoir auf. Das Gasreservoir ist ein Luftspeicher, der das Gasgemisch zur Durchführung einer Hypoxie-Behandlung und/oder eines Hypoxie-Trainings aufweist. Außerdem weist die Vorrichtung zur Bereitstellung einer hypoxischen Gasmischung eine Gasabgabevorrichtung auf. Die Gasabgabevorrichtung ist üblicherweise als Atemmaske ausgeführt, die ein Nutzer zur Durchführung einer Hypoxie-Behandlung und/oder eines Hypoxie-Trainings über den Atemöffnungen (Mund und Nase) trägt. Weiterhin weist die Vorrichtung zur Bereitstellung einer hypoxischen Gasmischung eine Zuleitung auf, die dafür geeignet und dafür vorgesehen ist, eine Gasmischung vom Gasreservoir zur Gasabgabevorrichtung zu leiten, und/oder einer Ableitung, die dafür geeignet und dafür vorgesehen ist, eine Gasmischung von der Gasabgabevorrichtung zum Gasreservoir zu leiten. Die Zuleitung und/oder Ableitung ist üblicherweise als flexibler Schlauch ausgeführt und verbindet die Gasabgabevorrichtung gasdicht mit dem Gasreservoir. Außerdem weist die Vorrichtung zur Bereitstellung einer hypoxischen Gasmischung eine Trägerelement auf, in dem erfindungsgemäß ein CO₂-Absorber anordenbar ist.

In einer Weiterbildung der Erfindung ist der CO₂-Absorber in dem Trägerelement platzierbar und wieder herausnehmbar ist. Optional ist das Trägerelement geeignet und auch dafür vorgesehen, den CO₂-Absorber als sog. Disposable aufzunehmen.

Ein Dispoable im Sinne der Erfindung ist eine Verpackungseinheit eines CO₂-Absorbers vorzugsweise mit einem festgelegten CO₂-Absorber-Gehalt. Der CO₂-Absorber kann hierbei beispielsweise selbst in einem Behältnis verpackt sein oder auch in loser Form vorliegen. In verpackter Form, hat das Disposable eine durch das Trägerelement der Vorrichtung zur Bereitstellung einer hypoxischen Gasmischung vorgegebene äußere Form.

Die offenbarungsgemäße Vorrichtung ist ein so genannter Pendelatmer, das heißt, ein und dieselbe Luft wird immer wieder ein- und ausgeatmet. Daher muss das sich anreichernde Kohlenstoffdioxid der Luft aus dem Atemkreislauf entfernt werden. Die normale Atemluft enthält 21 % Sauerstoff. Bei einem Atemzug werden der eingeatmeten Luft ca. 4 % Sauerstoff entzogen und durch eine entsprechende Menge ausgeatmeten Kohlenstoffdioxids (CO₂) ersetzt. Ein bestimmtes Volumen Luft kann also im Prinzip mehrmals "durchgeatmet" werden, bis sein Sauerstoffanteil erschöpft ist, allerdings reichert sich dabei das ausgeatmete Kohlenstoffdioxid in der Luft an. Außerdem bestehen physiologische Gefahren durch zu viel Kohlendioxid in der eingeatmeten Luft: ab 5 % kommt es zu Bewusstlosigkeit, ab 8 % über längere Zeit zum Tod.

Dazu weist die erfindungsgemäße Vorrichtung eine Trägerelement auf, in dem ein CO₂-Absorber angeordnet ist. Der CO₂-Absorber wird während der Hypoxie-Behandlung und/oder des Hypoxie-Trainings verbraucht und muss daher üblicherweise vor oder nach jeder Hypoxie-Behandlung und/oder Hypoxie-Training bzw. nach Verbrauch ausgetauscht werden. Das Trägerelement enthält den CO₂-Absorber und ist im Atemkreislauf der erfindungsgemäßen Vorrichtung angeordnet. Der CO₂-Absorber ist üblicherweise ein Gemisch aus Calciumhydroxid und Natriumhydroxid in fester Form (sog. Atemkalk). Während der Hypoxie-Behandlung und/oder des Hypoxie-Trainings durchströmt die Luft den Atemkalk, in welchem das Kohlenstoffdioxid erst an Natriumhydroxid gebunden wird, welches dann vom ebenfalls enthaltenen Calciumhydroxid, auch als gelöschter Kalk bekannt, wieder regeneriert wird.

Erfindungsgemäß ist der CO₂-Absorber in einem Behältnis angeordnet, wobei das Behältnis in dem Trägerelement angeordnet ist. Das Behältnis nimmt den pulverförmigen CO₂-Absorber auf, wobei das Behältnis als auswechselbare Kartusche ausgeführt ist, die vor bzw. nach der Hypoxie-Behandlung und/oder dem Hypoxie-Training gegen eine nicht verbrauchte Kartusche ausgetauscht werden kann.

In einer weiteren Ausgestaltung der Erfindung weist das Trägerelement eine Öffnung auf, durch die das Behältnis in das Trägerelement einführbar ist. Mittels der Öffnung ist das Behältnis vor bzw. nach der Hypoxie-Behandlung und/oder dem Hypoxie-Training austauschbar.

In einer weiteren Ausführung der Erfindung ist das Trägerelement verschließbar. Die Öffnung zum Austausch des Behältnisses ist verschließbar und insbesondere wieder öffenbar.

In einer Weiterbildung der Erfindung verfügt das Trägerelement über einen Körper und eine Klappe, wobei das Trägerelement mit der Klappe verschließbar ist. Die Klappe ist beweglich gegenüber dem Trägerelement angeordnet und öffnet bzw. verschließt das Trägerelement.

In einer weiteren Gestaltung der Erfindung ist zwischen der Klappe und dem Körper des Trägerelement eine Dichtung angeordnet, die dafür geeignet ist, das Trägerelement mit der Klappe luftdicht zu verschließen. Der im Behältnis angeordnete CO₂-Absorber nimmt daher ausschließlich Kohlendioxid aus der Atemluft des Nutzers auf und wird nicht durch Kohlendioxid aus der Umgebungsluft kontaminiert.

In einer weiteren Ausgestaltung der Erfindung ist das Behältnis an die Zuleitung und/oder die Ableitung derart anschließbar, dass die Gasmischung zur Durchführung der Hypoxie-Behandlung und/oder des Hypoxie-Trainings durch das Behältnis führbar ist. Damit wird sichergestellt, dass der im Behältnis angeordnete CO₂-Absorber das Kohlendioxid der Gasmischung entfernt.

In einem weiteren Aspekt der Erfindung ist das zwischen Behältnis und Zuleitung und/oder Ableitung eine Dichtung angeordnet, die verhindert, dass die Gasmischung aus dem Behältnis und der der Zu- und/oder Ableitung entweicht. Der im Behältnis angeordnete CO₂-Absorber nimmt daher ausschließlich Kohlendioxid aus der Atemluft des Nutzers auf und wird nicht durch Kohlendioxid aus der Umgebungsluft kontaminiert.

In einer weiteren Gestaltung der Erfindung ist das Behältnis derart in der Zu- und/oder Ableitung angeordnet, dass das Gasgemisch zwangsweise durch das Behältnis geleitet wird. Das Behältnis und der darin angeordnete CO₂-Absorber werden daher durch die Atemluft eines Nutzers beaufschlagt. Das Kohlendioxid der Gasmischung wird wirkungsvoll absorbiert.

In einer weiteren Ausführung der Erfindung weist die Vorrichtung eine Zuleitung und eine Ableitung auf. Getrennte Zuleitung und Ableitung gewährleistet, dass ein Nutzer nur von Kohlendioxid befreite Luft einatmet, die erfindungsgemäße Vorrichtung ist daher besonders sicher in der Anwendung.

Das offenbarungsgemäße (nicht beanspruchte) Verfahren zur Durchführung eines Hypoxie-Trainings weist zwei Verfahrensschritte auf: Im ersten Verfahrensschritt wird ein CO₂-Absorber in ein Trägerelement einer Vorrichtung zur Bereitstellung einer hypoxischen Gasmischung eingesetzt. Das Trägerelement ist im Atemkreislauf der Vorrichtung zur Bereitstellung einer hypoxischen Gasmischung angeordnet und ist geeignet, einen CO₂-Absorber aufzunehmen.

Im zweiten Verfahrensschritt wird die Gasausgabevorrichtung mit Mund und/oder Nase des Nutzers verbunden. Die Gasabgabevorrichtung ist üblicherweise als Atemmaske ausgeführt, die ein Nutzer während der Durchführung einer Hypoxie-Behandlung und/oder eines Hypoxie-Trainings über den Atemöffnungen (Mund und Nase) trägt. Dadurch wird gewährleistet, dass der Nutzer ausschließlich die hypoxische Gasmischung atmet, die die Vorrichtung zur Bereitstellung einer hypoxischen Gasmischung bereitstellt und in den Atemkreislauf einspeist.

In einer Weiterbildung der Erfindung ist der CO₂-Absorber in einem Behältnis angeordnet, wobei das Behältnis in dem Trägerelement angeordnet wird. Das Behältnis nimmt den pulverförmigen CO₂-Absorber auf, wobei das Behältnis als auswechselbare Kartusche ausgeführt ist, die vor bzw. nach der Hypoxie-Behandlung und/oder dem Hypoxie-Training gegen eine nicht verbrauchte ausgetauscht wird.

In einer weiteren Gestaltung der Erfindung wird das Behältnis an eine Zu- und/oder Ableitung der Vorrichtung zur Bereitstellung einer hypoxischen Gasmischung angeschlossen. Die Zuleitung ist dafür geeignet und dafür vorgesehen, eine Gasmischung von einem Gasreservoir zur Gasabgabevorrichtung zu leiten. Die Ableitung ist dafür geeignet und dafür vorgesehen, eine Gasmischung von der Gasabgabevorrichtung zu einem Gasreservoir zu leiten. Die Zuleitung und/oder Ableitung ist üblicherweise als flexibler Schlauch ausgeführt und verbindet die Gasabgabevorrichtung gasdicht mit dem Gasreservoir. Das Behältnis wird an die Zuleitung und/oder die Ableitung derart angeschlossen, dass die Gasmischung zur Durchführung der Hypoxie-Behandlung und/oder des Hypoxie-Trainings durch das Behältnis geführt wird. Damit wird sichergestellt, dass der im Behältnis angeordnete CO2-Absorber das Kohlendioxid der Gasmischung entfernt.

In einer weiteren Ausführung der Erfindung wird beim Anschließen des Behältnisses an eine Zu- und/oder Ableitung der Vorrichtung zur Bereitstellung einer hypoxischen Gasmischung eine luftdichte Verbindung zwischen dem Behältnis und der Zu- und/oder Ableitung hergestellt. Dadurch wird verhindert, dass die Gasmischung aus dem Behältnis und der der Zu- und/oder Ableitung entweicht. Der im Behältnis angeordnete CO₂-Absorber nimmt daher ausschließlich Kohlendioxid aus der Atemluft des Nutzers auf und wird nicht durch Kohlendioxid aus der Umgebungsluft kontaminiert.

In einer weiteren Ausbildung der Erfindung ist das Behältnis beim Einsetzen in die Vorrichtung zur Bereitstellung einer hypoxischen Gasmischung luftdicht versiegelt. Im Auslieferungszustand ist das Behältnis derart versiegelt, dass der im Behältnis angeordnete CO₂-Absorber nicht CO₂ aus der Umgebungsluft aufnimmt und damit schon vor dem Einsetzen in die Vorrichtung zur Bereitstellung einer hypoxischen Gasmischung verbraucht ist

In einer Weiterbildung der Erfindung wird das Behältnis an einer ersten Position geöffnet. In einem weiteren Aspekt der Erfindung wird das Behältnis an einer zweiten Position geöffnet. In einer weiteren Ausgestaltung der Erfindung sind die erste und die zweite Position am Behältnis gegenüberliegend angeordnet. Das Öffnen des Behältnisses öffnet eine Öffnung, durch die das Gasgemisch zur Durchführung eines Hypoxie-Trainings strömt.

In einer weiteren Gestaltung der Erfindung verbindet das Behältnis einen ersten Bereich der Zu- und/oder Ableitung mit einem zweiten Bereich der Zu- und/oder Ableitung. In einer weiteren Ausführung der Erfindung wird die erste und/oder die zweite Position mit dem Anschlussbereich des ersten und/oder zweiten Bereichs der Zu- und/oder Ableitung verbunden. Das Behältnis ist daher in der Zu- und/oder Ableitung angeordnet und der im Behältnis angeordnete CO₂-Absorber nimmt ausschließlich Kohlendioxid aus der Atemluft des Nutzers auf und wird nicht durch Kohlendioxid aus der Umgebungsluft kontaminiert.

Das Behältnis mit einem CO₂-Absorber zur Durchführung eines Hypoxie-Trainings ist erfindungsgemäß versiegelt. Insbesondere ist das Behältnis im Auslieferungszustand luftdicht versiegelt. Im Auslieferungszustand ist das Behältnis derart versiegelt, dass der im Behältnis angeordnete CO₂-Absorber nicht CO₂ aus der Umgebungsluft aufnimmt und damit schon vor dem Einsetzen in die Vorrichtung zur Bereitstellung einer hypoxischen Gasmischung verbraucht ist.

In einer Weiterbildung der Erfindung weist das Behältnis einen oder mehrere Öffnungsbereiche auf, wobei die Öffnungsbereiche dafür geeignet und dafür vorgesehen sind, das Behältnis in den Öffnungsbereichen zu öffnen In einem weiteren Aspekt der Erfindung weist der Öffnungsbereich eine Öffnung auf. Das Öffnen des Behältnisses öffnet die in den Öffnungsbereichen angeordnete Öffnungen, durch die das Gasgemisch zur Durchführung eines Hypoxie-Trainings strömt. Gegenüber der Umgebungsluft ist das Behältnis luftdicht ausgeführt.

In einer weiteren Gestaltung der Erfindung ist die Öffnung im Auslieferungszustand luftdicht versiegelt. Im Auslieferungszustand sind die Öffnungen des Behältnisses derart versiegelt, dass der im Behältnis angeordnete CO₂-Absorber nicht CO₂ aus der Umgebungsluft aufnimmt und damit schon vor dem Einsetzen in die Vorrichtung zur Bereitstellung einer hypoxischen Gasmischung verbraucht ist.

In einer weiteren Ausführung der Erfindung weist der Öffnungsbereich des Behältnisses eine Dichtfläche auf. In einer Weiterbildung weist die Dichtfläche eine Dichtung auf. Die Dichtung verhindert, dass die Gasmischung zur Durchführung eines Hypoxie-Trainings aus dem Behältnis und der der Zu- und/oder Ableitung entweicht, gleichzeitig wird verhindert, dass der im Behältnis angeordnete CO₂-Absorber durch Kohlendioxid aus der Umgebungsluft kontaminiert wird.

Ausführungsbeispiele der erfindungsgemäßen Vorrichtung zur Bereitstellung einer hypoxischen Gasmischung, des nicht beanspruchten Verfahrens zur Durchführung eines Hypoxie-Trainings sowie des Behältnisses mit einem CO2-Absorber zur Durchführung eines Hypoxie-Trainings sind in den Zeichnungen schematisch vereinfacht dargestellt und werden in der nachfolgenden Beschreibung näher erläutert.

Es zeigen:
- Fig. 1:: Erfindungsgemäße Vorrichtung zur Bereitstellung einer hypoxischen Gasmischung, eine Zu- und Ableitung
- Fig. 2:: Erfindungsgemäße Vorrichtung zur Bereitstellung einer hypoxischen Gasmischung, Zu- und Ableitung getrennt, Trägerelement in Ableitung
- Fig. 3:: Erfindungsgemäße Vorrichtung zur Bereitstellung einer hypoxischen Gasmischung, Zu- und Ableitung getrennt, Trägerelement in Zuleitung
- Fig. 4 a:: Seitenansicht eines Trägerelements

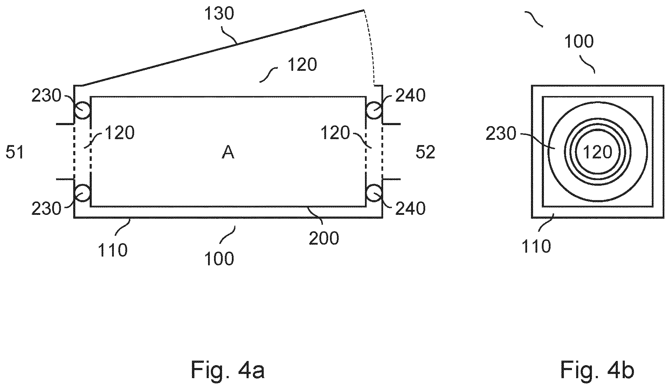

- Fig. 4 b:: Frontansicht eines Trägerelements

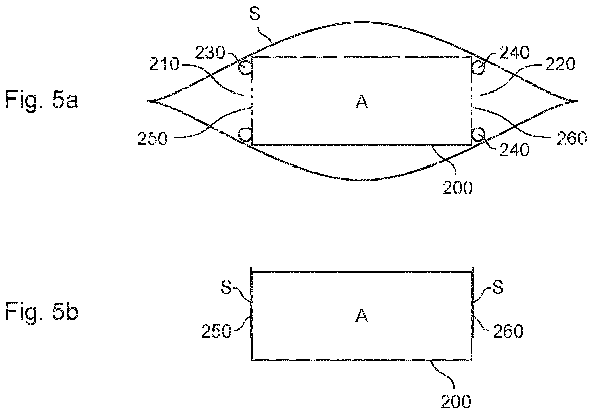

- Fig. 5 a:: Ansicht eines versiegelten Behältnisses
- Fig. 5 b:: Weitere Ansicht eines versiegelten Behältnisses

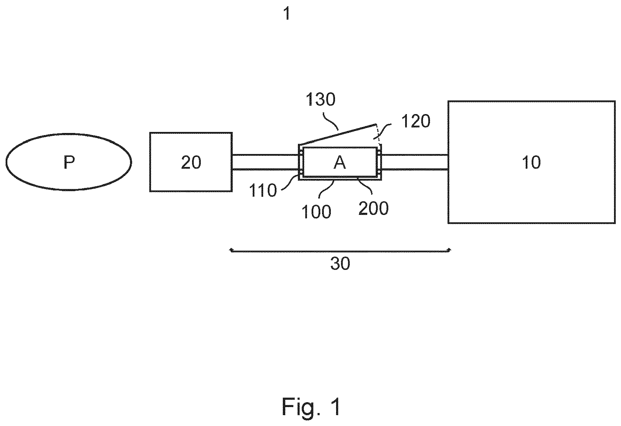

Fig. 1 zeigt eine Ansicht einer Vorrichtung 1 zur Durchführung einer Hypoxie-Behandlung und/oder eines Hypoxie-Trainings. Die Vorrichtung 1 weist die Gasabgabevorrichtung 20 auf, die als Atemmaske ausgeführt ist und vom Nutzer P während der Hypoxie-Behandlung und/oder des Hypoxie-Trainings über den Atemöffnungen (Mund und Nase) getragen wird. Die Vorrichtung 1 weist außerdem das Gasreservoir 10 auf. Gasreservoir 10 und Gasabgabevorrichtung 20 sind miteinander über die flexibel ausgeführte Gasleitung 30 gasdicht miteinander verbunden. Die Gasleitung 30 weist das Trägerelement 100 zur Aufnahme des CO₂-Absorbers A auf. In allen hier gezeigten Ausführungsbeispielen ist der CO₂-Absorber A ein Gemisch aus Calciumhydroxid Ca(OH)₂ und Natriumhydroxid NaOH, auch als Atemkalk bezeichnet. Das Trägerelement 100 ist in der Gasleitung 30 derart angeordnet, dass das Gasgemisch während der Hypoxie-Behandlung und/oder des Hypoxie-Trainings durch das Trägerelement 100 geführt wird. Das Trägerelement 100 weist den Körper 110 und die Öffnung 120 auf. Die Öffnung 120 ist mittels der Klappe 130 öffenbar und wieder verschließbar.

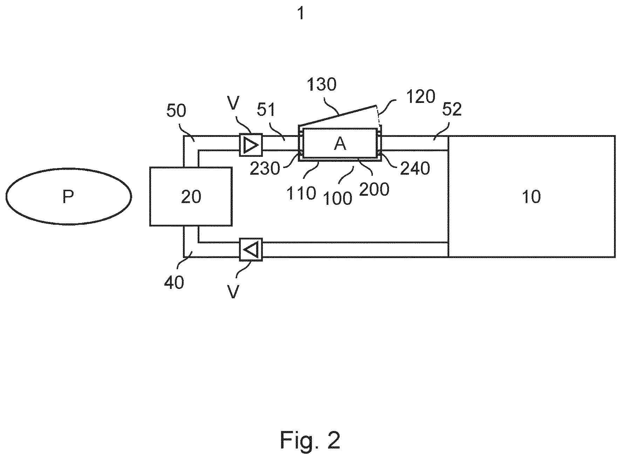

Eine bevorzugte Ausführungsform der erfindungsgemäßen Vorrichtung 1 zur Durchführung einer Hypoxie-Behandlung und/oder eines Hypoxie-Trainings zeigt Fig. 2. Die Vorrichtung 1 weist ebenfalls die Gasabgabevorrichtung 20 auf. Die Gasabgabevorrichtung 20 ist mit dem Gasreservoir 10 über eine flexible Zuleitung 40 und eine ebenfalls flexible Ableitung 50 verbunden. Zuleitung 40 und Ableitung 50 weisen jeweils ein Einwege-Ventil V auf. Die Einwege-Ventile V gewährleisten, dass der Nutzer P nur die Luft aus der Zuleitung 40 einatmet und verhindern damit, dass beim Einatmen der Nutzer P CO₂-haltige Luft aus der Ableitung 50 einatmet.

In diesem Ausführungsbeispiel weist die Ableitung 50 das Trägerelement 100 zur Aufnahme des CO₂-Absorbers A auf. Das Trägerelement 100 weist den Körper 110 und die Öffnung 120 auf, die mittels der Klappe 130 öffenbar und wieder luftdicht verschließbar ist. Die Klappe 130 weist dazu eine geeignete Dichtung 140 auf. Die Ableitung 50 weist den ersten Bereich 51 und den zweiten Bereich 52 auf, die durch das Trägerelement 100 luftdicht miteinander verbunden sind.

Der CO₂-Absorber A ist in einem Behältnis 200 angeordnet (s. Fig. 4, Fig. 5). Das Behältnis 200 wird zur Durchführung der Hypoxie-Behandlung und/oder des Hypoxie-Trainings vor Beginn der Hypoxie-Behandlung und/oder des Hypoxie-Trainings in das Trägerelement 100 bei geöffneter Klappe 130 eingeführt. Das Trägerelement 100 wird mittels der Klappe 130 luftdicht verschlossen und die Gasabgabevorrichtung 20 (Atemmaske) wird mit Mund und Nase des Nutzers P luftdicht verbunden.

Danach beginnt die eigentliche Hypoxie-Behandlung und/oder das Hypoxie-Training, wobei der Nutzer P ein und dieselbe Luft wird immer wieder ein- und ausatmet. Der CO₂-Absorber A verhindert ein Ersticken. Die Vorrichtung 1 weist dazu Messgeräte auf, die den gesundheitlichen Zustand des Nutzers P während der Hypoxie-Behandlung und/oder des Hypoxie-Trainings permanent überwachen. Zur Anwendung kommt insbesondere ein Pulsoximeter, mit dem die Herzfrequenz und der Sauerstoffgehalt des Blutes überwacht werden. Die Messgeräte sind mit einer Steuereinheit verbunden, die bei auftretenden Komplikationen, z.B. zu niedrigem Sauerstoffgehalt im Blut des Nutzers, ein Alarmsignal gibt, damit die Hypoxie-Behandlung und/oder das Hypoxie-Training sofort abgebrochen wird.

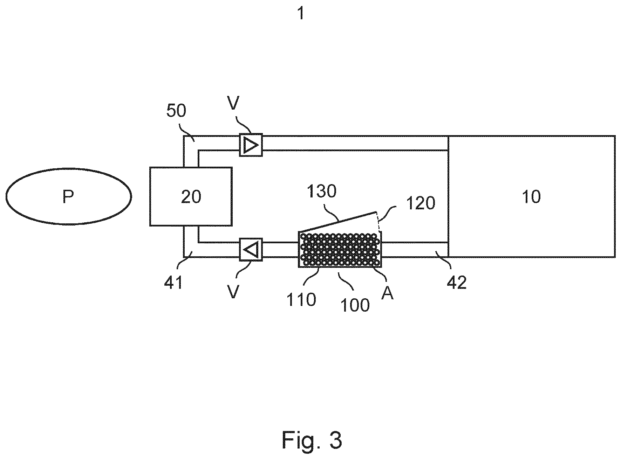

Fig. 3 zeigt ein weiteres Ausführungsbeispiel der erfindungsgemäßen Vorrichtung 1 zur Durchführung einer Hypoxie-Behandlung und/oder eines Hypoxie-Trainings. Die Vorrichtung 1 weist ebenfalls die Gasabgabevorrichtung 20 auf. Die Gasabgabevorrichtung 20 ist mit dem Gasreservoir 10 über eine flexible Zuleitung 40 und eine ebenfalls flexible Ableitung 50 verbunden. Zuleitung 40 und Ableitung 50 weisen jeweils ein Einwege-Ventil V auf. In diesem Ausführungsbeispiel ist das Trägerelement 100 mit Körper 110, Klappe 130 und Öffnung 120 in der Zuleitung 40 angeordnet, wobei der erste Bereich 41 der Zuleitung 40 und der zweite Bereich 42 der Zuleitung 40 jeweils mit dem Trägerelement 100 luftdicht verbunden sind.

Der CO₂-Absorber A ist in diesem Ausführungsbeispiel nicht in einem Behältnis 200 angeordnet, sondern ist als pulverförmiges Granulat in dem Trägerelement 100 derart angeordnet, dass bei Durchführung einer Hypoxie-Behandlung und/oder eines Hypoxie-Trainings die Atemluft durch den CO₂-Absorber A zwangsweise geleitet wird.

Ein Ausführungsbeispiel eines mit dem Behältnis 200 versehenen Trägerelements 100 im Auslieferungszustand zeigt Fig. 4. Das Trägerelement 100 weist eine quaderförmige Form auf (Fig. 4 a), die durch den Körper 110 zur Aufnahme des Behältnisses 200 gebildet wird. Mittels der Klappe 130 ist der Körper 110 öffenbar bzw. luftdicht verschließbar. Zwischen Klappe 130 und dem Trägerelement 100 bzw. dem Körper 110 ist dazu ein Dichtung 140 angeordnet. Die Öffnung 120 ist derart bemessen, dass das Behältnis 200 in den Körper 110 eingeführt bzw. wieder entnommen werden kann.

An den zueinander gegenüberliegenden Stirnseiten (Fig. 4 b) ist das Trägerelement 100 luftdicht an einer Seite mit dem ersten Bereich 41, 51 der Zuleitung 40 bzw. der Ableitung 50 verbunden, an der gegenüberliegenden Seite luftdicht mit dem zweiten Bereich 42, 52 der Zuleitung 40 bzw. der Ableitung 50. Das Trägerelement 100 weist dazu an den Innenseiten der Stirnseiten jeweils eine kreisförmige Dichtung 230, 240 auf, das Behältnis 200 weist korrespondierend ausgeführte Dichtflächen jeweils an den Außenseiten der Stirnflächen auf. Die Dichtungen 230, 240 können alternativ auch auf dem Behältnis 200 angeordnet sein (s. Fig. 5 a), das Trägerelement 100 benötigt dann keine derartigen Dichtungen 230, 240. Das Gasgemisch zur Durchführung einer Hypoxie-Behandlung und/oder eines Hypoxie-Trainings wird daher zwangsweise durch das Behältnis 200 geleitet.

Fig. 5 zeigt Ausführungsbeispiele von Behältnissen 200 im Auslieferungszustand, die den CO2-Absorber A enthalten. Der CO₂-Absorber A ist Atemkalk, ein Gemisch aus Calciumhydroxid Ca(OH)₂ und Natriumhydroxid NaOH. Das Behältnis 200 ist in diesem Ausführungsbeispiel zylinderförmig und weist an den Stirnseiten jeweils einen Öffnungsbereich 210, 220 auf, an denen das Behältnis 200 öffenbar ist. Ein Öffnungsbereich 210, 220 weist jeweils eine Öffnung 250, 260 auf.

Das Behältnis 200 kann an den Stirnseiten Dichtflächen mit jeweils einer kreisförmigen Dichtung 230, 240 aufweisen (Fig. 5 a). In diesem Fall weist das Trägerelement 100 selbst keine entsprechenden Dichtungen auf, um das Behältnis 200 derart luftdicht mit Ableitung 40 oder Zuleitung 40 zu verbinden. Alternativ weist das Trägerelement 100 Dichtungen 230, 240 auf (s. Fig. 4). In diesem Fall weist das Behältnis 200 selbst keine Dichtungen auf (Fig. 5 b).

Um eine Beladung des CO₂-Absorbers A mit CO₂ aus der Umgebungsluft zu verhindern, ist das Behältnis 200 im Auslieferungszustand versiegelt. Die Versiegelung S kann als Kunststofffolie ausgeführt das Behältnis 200 vollständig umschließen (Fig. 5 a). Zur Entnahme des Behältnisses 200 öffnet ein Nutzer P die Versiegelung S und entfernt diese. Alternativ sind nur die Öffnungsbereiche 210, 220 und insbesondere die Öffnungen 250, 260 selbst mit jeweils einer Versiegelung S versehen. Zum Einsetzen des Behältnisses 200 in das Trägerelement 100 entfernt ein Nutzer P die Versiegelung S.

### BEZUGSZEICHENLISTE

- 1: Vorrichtung zur Bereitstellung einer hypoxischen Gasmischung
- 10: Gasreservoir
- 20: Gasabgabevorrichtung / Beatmungsmaske
- 30: Gasleitung
- 40: Zuleitung
- 41: Erster Bereich der Zuleitung
- 42: Zweiter Bereich der Zuleitung
- 50: Ableitung
- 51: Erster Bereich der Ableitung
- 52: Zweiter Bereich der Ableitung
- 100: Trägerelement
- 110: Körper
- 120: Öffnung
- 130: Klappe
- 140: Dichtung zwischen Körper und Klappe
- 200: Behältnis
- 210, 220: Öffnungsbereich
- 230, 240: Dichtung zwischen Öffnungsbereich und Zuleitung / Ableitung
- 250, 260: Öffnung
- A: CO₂-Absorber
- P: Nutzer
- S: Versiegelung des Behältnisses
- V: Einwege-Ventil

## Patentansprüche

1. Vorrichtung (1) zur Bereitstellung einer hypoxischen Gasmischung mit:
• einem Gasreservoir (10),
• eine Gasabgabevorrichtung (20),
• einer Zuleitung (40), die dafür geeignet und dafür vorgesehen ist, eine Gasmischung vom Gasreservoir (10) zur Gasabgabevorrichtung (20) zu leiten,
und/oder
einer Ableitung (50), die dafür geeignet und dafür vorgesehen ist, eine Gasmischung von der Gasabgabevorrichtung (20) zum Gasreservoir (10) zu leiten und
• einem Trägerelement (100),
wobei in dem Trägerelement (100) ein CO₂-Absorber (A) anordenbar ist,
wobei die Vorrichtung (1) ein Behältnis (200) aufweist,
wobei der CO₂-Absorber (A) in dem Behältnis (200) angeordnet ist,
wobei das Behältnis (200) in dem Trägerelement (100) anordenbar ist, sodass vor bzw. nach der Hypoxie-Behandlung und/oder dem Hypoxie-Training ein verbrauchtes Behältnis (200) gegen ein nicht verbrauchtes Behältnis (200) ausgetauscht werden kann.

2. Vorrichtung (1) zur Bereitstellung einer hypoxischen Gasmischung nach Anspruch 1,
**dadurch gekennzeichnet, dass**
das Trägerelement (100) eine Öffnung (120) aufweist, durch die das Behältnis (200) in das Trägerelement (100) einführbar ist.

3. Vorrichtung (1) Bereitstellung einer hypoxischen Gasmischung nach Anspruch 1 oder 2,
**dadurch gekennzeichnet, dass**
das Trägerelement (100) verschließbar ist,
wobei das Trägerelement (100) über einen Körper (110) und eine Klappe (130) verfügt wobei das Trägerelement (100) mit der Klappe verschließbar ist,
wobei zwischen der Klappe (130) und dem Körper (110) des Trägerelements (100) eine Dichtung (140) angeordnet ist, die dafür geeignet ist, das Trägerelement (100) mit der Klappe (130) luftdicht zu verschließen.

4. Vorrichtung (1) Bereitstellung einer hypoxischen Gasmischung nach Anspruch 2 oder 3,
**dadurch gekennzeichnet, dass**
das Behältnis (200) an die Zuleitung (40) und/oder die Ableitung (50) derart anschließbar ist, dass die Gasmischung durch das Behältnis (200) führbar ist, wobei das zwischen Behältnis (200) und Zuleitung (40) und/oder Ableitung (50) eine Dichtung (230, 240) angeordnet ist, die verhindert, dass die Gasmischung aus dem Behältnis (200) und der Zuleitung (40) und/oder Ableitung (50) entweicht,
wobei das Behältnis (200) derart in der Zuleitung (40) und/oder Ableitung (50) angeordnet ist, dass das Gasgemisch zwangsweise durch das Behältnis (200) geleitet wird.

5. Vorrichtung (1) Bereitstellung einer hypoxischen Gasmischung nach einem oder mehreren der vorhergehenden Ansprüche,
**dadurch gekennzeichnet, dass**
die Vorrichtung (1) die Zuleitung (40) und die Ableitung (50) aufweist.

## Claims

1. A device (1) for providing a hypoxic gas mixture, comprising:
• a gas reservoir (10),
• a gas dispensing device (20),
• a supply line (40) adapted and configured to convey a gas mixture from the gas reservoir (10) to the gas dispensing device (20), and/or an outlet line (50) suitable and intended for conveying a gas mixture from the gas dispensing device (20) to the gas reservoir (10), and
• a support element (100),
wherein a CO₂ absorber (A) can be arranged in the support element (100), wherein the device (1) comprises a container (200),
wherein the CO₂ absorber (A) is arranged in the container (200),
wherein the container (200) can be arranged within the support element (100), so that, before or after hypoxia treatment and/or hypoxia training, a used container (200) can be replaced with an unused container (200).

2. A device (1) for providing a hypoxic gas mixture according to claim 1,
**characterized in that**
the carrier element (100) has an opening (120) through which the container (200) can be inserted into the carrier element (100).

3. A device (1) for supplying a hypoxic gas mixture according to claim 1 or 2,
**characterized in that**
the support element (100) is closeable,
wherein
the carrier element (100) comprises a body (110) and a flap (130),
wherein the carrier element (100) can be closed with the flap,
wherein a seal (140) is arranged between the flap (130) and the body (110) of the carrier element (100), which seal is suitable for closing the carrier element (100) with the flap (130) in an airtight manner.

4. A device (1) for supplying a hypoxic gas mixture according to claim 2 or 3,
**characterized in that**
the container (200) can be connected to the supply line (40) and/or the discharge line (50) such that the gas mixture can be passed through the container (200),
wherein a seal (230, 240) is arranged between the container (200) and the supply line (40) and/or the discharge line (50), which prevents the gas mixture from escaping from the container (200) and the supply line (40) and/or the discharge line (50),
wherein the container (200) is arranged in the supply line (40) and/or discharge line (50) such that the gas mixture is forced through the container (200).

5. Apparatus (1) for supplying a hypoxic gas mixture according to one or more of the preceding claims,
**characterized in that**
the device (1) comprises the supply line (40) and the discharge line (50).

## Revendications

1. Dispositif (1) pour fournir un mélange gazeux hypoxique comprenant :
• un réservoir de gaz (10),
• un dispositif de distribution de gaz (20),
• une conduite d'amenée (40) adaptée et destinée à acheminer un mélange gazeux du réservoir de gaz (10) vers le dispositif de distribution de gaz (20), et/ou
une conduite d'évacuation (50) adaptée et destinée à acheminer un mélange gazeux du dispositif de distribution de gaz (20) vers le réservoir de gaz (10) et
• un élément de support (100),
dans lequel un absorbeur de CO₂ (A) peut être disposé dans l'élément de support (100),
dans lequel le dispositif (1) présente un récipient (200),
dans lequel l'absorbeur de CO₂ (A) peut être disposé dans le récipient (200),
dans lequel le récipient (200) peut être disposé dans l'élément de support (100),
de sorte que, avant ou après le traitement de l'hypoxie et/ou l'entraînement à l'hypoxie, un récipient usagé (200) puisse être échangé contre un récipient non utilisé (200).

2. Dispositif (1) pour fournir un mélange gazeux hypoxique selon la revendication 1,
**caractérisé en ce que**
l'élément de support (100) présente une ouverture (120) à travers laquelle le récipient (200) peut être inséré dans l'élément de support (100).

3. Dispositif (1) pour fournir un mélange gazeux hypoxique selon la revendication 1 ou 2,
**caractérisé en ce que**
l'élément de support (100) est scellable,
dans lequel l'élément de support (100) comporte un corps (110) et un volet (130),
dans lequel l'élément de support (100) peut être scellé avec le volet,
dans lequel un joint (140) est disposé entre le volet (130) et le corps (110) de l'élément de support (100), ce qui convient pour sceller l'élément de support (100) avec le volet (130) de manière étanche à l'air.

4. Dispositif (1) pour fournir un mélange gazeux hypoxique selon l'une ou plusieurs des revendications 2 ou 3,
**caractérisé en ce que**
le récipient (200) peut être relié à la conduite d'amenée (40) et/ou à conduite d'évacuation (50) de manière à ce que le mélange gazeux puisse être guidé à travers le récipient (200),
dans lequel un joint (230, 240) est disposé entre le récipient (200) et la conduite d'amenée (40) et/ou la conduite d'évacuation (50), empêchant ainsi le mélange gazeux de s'échapper du récipient (200) et de la conduite d'amenée (40) et/ou de la conduite d'évacuation (50),
dans lequel le récipient (200) est disposé dans la conduite d'amenée (40) et/ou la conduite d'évacuation (50) de telle sorte que le mélange gazeux soit guidé de manière forcée à travers le récipient (200).

5. Dispositif (1) pour fournir un mélange gazeux hypoxique selon l'une ou plusieurs des revendications précédentes,
**caractérisé en ce que**
le dispositif (1) présente la conduite d'amenée (40) et la conduite d'évacuation (50).
